# EUROPEAN PATENT APPLICATION

(11) **EP 3 251 683 A1**
(43) Date of publication of application: **06.12.2017**
(21) Application number: 15879428.9
(22) Date of filing: 30.01.2015
(51) Int. Cl.: A61K 36/46, A61K 31/7048, A61K 31/075, A61K 9/08, A61K 9/14, A61K 9/20, A61K 9/48, A61P 17/06, A61K 127/00

(54) **EUCOMMIA LEAF EXTRACT, AND PREPARATION METHOD AND USE THEREOF**

(71) Applicant: Sichuan Jiuzhang Biological Science And Technology Co., Ltd, High-tech District Chengdu Sichuan 610041 (CN)
(72) Inventor: ZHANG, Jie, Chengdu Sichuan 610041 (CN); HUANG, Wang, Chengdu Sichuan 610041 (CN); ZHANG, Liang, Chengdu Sichuan 610041 (CN); HUANG, Jun, Chengdu Sichuan 610041 (CN)
(74) Representative: Herzog, Fiesser & Partner Patentanwälte PartG mbB
(86) International application number: PCT/CN2015/071956
(87) International publication number: WO 2016/119220

(57) **Abstract**

Disclosed are a eucommia leaf extract, and preparation method and use thereof. The extract is a liquid of the eucommia leaf or an ethyl alcohol extract, and has chlorogenic acid of a content not less than 9%, geniposidic acid of a content not less than 1% and aucubin of a content not less than 1%. The extract has a good effect on psoriasis.

## Description

### Technical field

The present invention relates to the eucommia leaf extract, together with its preparative method and uses thereof.

### Background art

Psoriasis, commonly called "oxhide lichen", is a common and recurrent skin disease with chronic inflammation, and characterized by mica-like scale, red membrane sign, punctuate hemorrhage, with an incidence rate of about 0.123% in China. In recent years, the incidence of psoriasis shows tendency to ascend, and in the initial stage, psoriasis has an apparent seasonality, and presents severity in winter and light in summer. At present, the cause of disease is unknown, and effective therapeutic method is deficient.

Currently, psoriasis includes four types, i.e. psoriasisvulgaris, psoriasis arthropathica, erythrodermapsoriaticum, and psoriasis pustulosa, in which psoriasisvulgarisaccounts for above 99% of psoriasis. Psoriasisvulgaris is an ordinary psoriasis, while the other three types are called specific psoriasis. According to experience summarization of famous doctors of traditional Chinese medicine in successive dynasties, psoriasisvulgaris is generally classified as four types, i.e. blood-heat type (blood-heat and wind-dryness type), blood-deficiency type (blood-deficiency and wind-dryness type), blood-dryness type, blood stasis type (thedermalhematomalikelytype); for special psoriasis, pustule type is generally discriminated as sepsis type (damp-heat and accumulated toxin type), and erythroderma type is poison and heat type (blood-heat toxic pattern), and joint type is cold-dampness or rheumatic arthralgia type.

Primary clinical manifestations of psoriasis vulgaris include white scale, shining thin film and punctuate hemorrhage. Main pathologic manifestations include: parakeratosis, visible microabscess (Munro's abscess) arising from neutrophilic granulocyte, granular layer thinning or vanishing; acanthosis, trochanterellus extension; blood vessels in dermal papilla distort and expand, and vessel wall slightly thickens; superior part of dermis is infiltrated by inflammatory cells of from mild to moderate. Psoriasis vulgaris is more common, and approximately occupied above 90%.

Psoriasis pustulosa is a type of psoriasis having more severe pathogenetic condition and divided into generalized psoriasis and localized psoriasis. In clinical, it is characterized by generalized erythema spreading whole body, as well as sterile pustules with a miliarysize, and frequently accompanied by hyperpyrexia and increase of white blood cell and hypoproteinemia, even can threaten to life. This type is rarer in clinic, and accounts for about 0.77% of patients with psoriasis.

Tissue pathology of psoriasis is that epidermic keratinocytes can not fully matured, and spaces between cell bundles fill with air and refract ray to form silver mica-like scale, as seen in clinical; intradermal papilledema swells and intrudes into epidermis, getting close to corneum layer, and in clinical, scraping scales may impair papilla blood vessels and produce punctuate hemorrhage. Medical doctors consider that development of psoriasis is mainly related to functional disorder of autoimmunity, metabolism and endocrine, and climatic change, labile mood, infection, trauma and so on are causative factors.

Pathogenesis of psoriasis is complex, and at present, it is not completely be interpreted. The pathogenesis may roughly be summarized as follows: on the basis of certain genetic backgrounds, various causative agents stimulate the immune system of organisms, and cause functional disorders of immune system focusing on T cells. Inflammatory cells migrate to epidermis and locally infiltrate, resulting in part inflammation and paraplasm of keratinocytes, and finally resulting in occurrence of pathologic change of psoriasis.

Psoriasis is a common chronic inflammatory skin disease, with hyperplasia of keratinocytes, infiltration of inflammatory cells, and neovascularization as main pathologic changes, and is a commonly encountered disease and a frequently occurring disease in department of dermatology. Pathogenesis of this disease is unknown, and its treatment is difficult, and it can not be completely eradicated. Currently, western medicaments tretinoin and immunosuppressive agents are used to treat this disease, especially possessing advantages in controlling psoriasis activity, but they have problems including more adverse reactions and high recurrence rate following drug withdrawal, etc.

Eucommina leaves are the dried leaves of plant *Eucommiaulmoides* Oliv. of Eucommiaceae, with slight xin, warm, and channel tropism of the liver and kidney. Eucommina leaves can tonify the liver and kidney, as well as strengthen bones and muscles. The leaves are used for treatment of insufficiency of the liver and kidney, dizziness, sore and pain in loin and legs. There areno reports on uses of the eucommina leaf extract in treating psoriasis.

### Contents of the invention

The technical solution of the present invention provides the eucommia leaf extract, together with its preparative method and uses thereof.

Aeucommia leaf extract is the water or ethanolic extract of eucommia leaves, in which the content of chlorogenic acid is not less than 9%, the content of geniposidic acid is not less than 1%, and the content of aucubin is not less than 1%.

Preferably, in the eucommia leaf extract, the content of chlorogenic acid is 9.38%-39.11%, the content of geniposidic acid is 1.03%-10.33%, and the content of aucubin is 1.17%-10.68%.

More preferably, in the eucommia leaf extract, the content of chlorogenic acid is 10.21%-32.37%, the content of geniposidic acid is 1.03%-9.38%, and the content of aucubin is 1.17%-9.56%.

Further more preferably, in the eucommia leaf extract, the content of chlorogenic acid is 10.21% or 32.37%, the content of geniposidic acid is 1.03% or 2.24%, and the content of aucubin is 1.24% or 2.07%.

In which, the eucommia leaf extract is prepared by the following method:
The eucommia leaves are extracted with water or 30-80% ethanol for 1-2 times, 1-2 h for each time. The extracting solution or its concentrated solution is absorbed by polystyrene-type macroporous absorption resins, followed by elution with gradient ethanol of 10-90%, and the eluent is collected, combined, concentrated and dried, to provide the extract.

Preferably, the eucommia leaf extract is prepared by the following methods:
The eucommia leaves are extracted twice with 60% ethanol (using 15 times the volume of leaves) at 70 °C, 1 h for each time. The extract is combined and concentrated to recovery ethanol, and the concentrated solution is saturatedly absorbed by D-101 macroporous absorption resins, and eluted with gradient ethanol of 10%, 30%, 70%, and 90%, to collect elution. 10%elution and 30% elution are combined, or 70% elution and 90% elution are combined, and then concentrated and dried, respectively, to provide the extract;
Alternatively, the eucommia leaves are extracted twice with 30% ethanol (using 12 times the volume of leaves) at 60 °C, 2 h for each time. The extract is combined and concentrated to recovery ethanol, and the concentrated solution is saturatedly absorbed by HPD-100 macroporous absorption resins, and eluted with gradient ethanol of 10%, 30%, 70%, and 90%, to collect elution. 70% EtOH elution and 90% EtOH elution are combined, concentrated and dried, to provide the extract, or 30% EtOH elution is concentrated and dried, to provide the extract;
Alternatively, the eucommia leaves are extracted once with water (using 20 times the volume of leaves) at 80 °C, 1.5 h for each time. The extract is saturatedly absorbed by XAD-16 macroporous absorption resins, and successively eluted with gradient ethanol of 20% and 60%, to respectively collect 20% elution and 60% elution, which are concentrated and dried, to provide the extract;
Alternatively, the eucommia leaves are extracted twice with water (using 12 times the volume of leaves) at 60 °C, 2 h for each time. The extract is saturatedly absorbed by AB-8 macroporous absorption resins, and successively eluted with gradient ethanol of 10% and 90%, to respectively collect 10% elution and 90% elution, which are concentrated and dried, to provide the extract.

The preparative method of eucommia leaf extract includes the following steps: the eucommia leaves are extracted with water or 30-80% EtOH for 1-2 times, and the extraction or its concentrated solution are absorbed by polystyrene-type macroporous absorption resins, followed by elution with gradient ethanol of 10-90%, and the eluent is collected, combined, concentrated and dried, to provide the extract.

Preferably, the method includes the following steps:
The eucommia leaves are extracted twice with 60% ethanol (using 15 times the volume of leaves) at 70 °C, 1 h for each time. The extract is combined and concentrated to recovery ethanol, and the concentrated solution is saturatedly absorbed by D-101 macroporous absorption resins, and successively eluted with gradient ethanol of 10%, 30%, 70%, and 90%, to collect elution. 10% elution and 30% elution are combined, and 70% elution and 90% elution are combined, and then concentrated and dried, respectively, to provide the extract;
Alternatively, the eucommia leaves are extracted twice with 30% ethanol (using 12 times the volume of leaves) at 60 °C, 2 h for each time. The extract is combined and concentrated to recovery ethanol, and the concentrated solution is saturatedly absorbed by HPD-100 macroporous absorption resins, and eluted with gradient ethanol of 10%, 30%, 70%, and 90%, to collect elution. 70% EtOH elution and 90% EtOH elution are combined, concentrated and dried, to provide the extract, or 30% EtOH elution is concentrated and dried, to provide the extract;
Alternatively, the eucommia leaves are extracted once with water (using 20 times the volume of leaves) at 80 °C, 1.5 h for each time. The extract is saturatedly absorbed by XAD-16 macroporous absorption resins, and successively eluted with gradient ethanol of 20% and 60%, to respectively collect 20% elution and 60% elution, which are concentrated and dried, to provide the extract;
Alternatively, the eucommia leaves are extracted twice with water (using 12 times the volume of leaves) at 60 °C, 2 h for each time. The extract is saturatedly absorbed by AB-8 macroporous absorption resins, and successively eluted with gradient ethanol of 10% and 90%, to respectively collect 10% elution and 90% elution, which are concentrated and dried, to provide the extract.

The present invention also provides the uses of the eucommia leaf extract in the preparation of medicaments for treatment of psoriasis.

Wherein, said medicaments are those treating psoriasisvulgaris and psoriasis pustulosa.

The present invention further provides a medicament for treatment of psoriasis, and it is prepared using the above-mentioned eucommia leaf extract as active ingredient, together with pharmaceutically acceptable adjuvants or auxiliary ingredients.

Wherein, said medicament is oral preparations, external preparations.

Wherein, said oral preparations are tablets, granules, pulvis, pills, oral liquid, and capsules.

Psoriasis is a chronic inflammatory and proliferative skin disease with immunologic abnormality, that is determined by polygenic inheritance and induced by many environmental factors. Worldwide, the prevalence in natural population is 0.1%-3%, while the prevalence in China is 0.123%, and the annual incidence is 0.1%. Although this disease does not endanger people's lives, the course of disease is long, and it is stubbornly and intractable. Serious person may develop a disabling psoriatic arthritis, erythrodermapsoriaticum, and even involve eyes, liver, kidney, cardiovascular, lung and similar organs, thus severely affect life quality of patients. Some patients can have psychological disorders such as blushing, stress, anxiety, depress, etc., and are not willing to take part in social activities, which affect their daily life, create deep suffering for patients' body and psychology. Because of recurrent attacks of this disease, repeat treatment is needed, and brought a heavy economic burden to patients and their family. Currently, psoriasis can not be completely eradicated. Except for traditional Chinese medicines, traditional treatment mostly focuses on immunosuppressive agents and retinoids, and long-term administration of these medicaments may easily produce seriously adverse effects including dependence and damage on liver and kidney.

The eucommia leaf extract, as a traditional Chinese medicine extract, has a little side effect, and can be used for a long time, but do not cause toxic and side action. The extract is safe and effective, and can improve the life quality of patients, especially with a significant therapeutic effect on psoriasis. Thus, for treatment of psoriasis, the eucommia leaf extract has a wide application prospects.

Obviously, based on above contents of the present invention, without departing from above basic technical spirit of the present invention, various additional modifications, alternations and changes can also be made by common technical knowledge and commonly-used means in the field.

Hereinafter, above contents of the present invention can further be illustrated with reference to specific examples. But it should not be understood that above subject scope of the present invention is limited to the following examples. The technology that can be realized based on above contents of the present invention should all be within the scope of the present invention.

### Examples

### Example 1 Preparation of eucommia leaf extract according to the present invention

The eucommia leaves were extracted twice with 60% ethanol (using 15 times the volume of leaves) at 70 °C, 1 h for each time. The extract was combined and concentrated to recovery ethanol, and the concentrated solution was saturatedly absorbed by D-101 macroporous absorption resins, and eluted with gradient ethanol of 10%, 30%, 70%, and 90%, to collect elution. 10%elution and 30% elution were combined, concentrated and dried, to provide the extract sample 1 (Content: chlorogenic acid = 11.27%,geniposidic acid = 1.87%,aucubin=1.17%); 70%elution and 90% elution were combined, and then concentrated and dried, to provide the extract sample 2 (Content: chlorogenic acid = 32.37%, geniposidic acid = 2.24%, aucubin = 2.07%).

### Example 2 Preparation of eucommia leaf extract according to the present invention

The eucommia leaves were extracted twice with 30% ethanol (using 12 times the volume of leaves) at 60 °C, 2 h for each time. The extract was combined and concentrated to recovery ethanol, and the concentrated solution was saturatedly absorbed by HPD-100 macroporous absorption resins, and eluted with gradient ethanol of 10%, 30%, 70%, and 90%, to collect elution. 70% EtOH elution and 90% EtOH elution were combined, concentrated and dried, to provide the extract sample 1 (Content: chlorogenic acid = 11.24%, geniposidic acid = 9.38%, aucubin = 9.56%), or 30% EtOH elution is concentrated and dried, to provide the extract sample 2 (Content: chlorogenic acid = 38.55%, geniposidic acid = 1.18%, aucubin = 1.36%).

### Example 3 Preparation of eucommia leaf extract according to the present invention

The eucommia leaves were extracted once with water (using 20 times the volume of leaves) at 80 °C, 1.5 h for each time. The extract was saturatedly absorbed by XAD-16 macroporous absorption resins, and successively eluted with gradient ethanol of 20% and 60%, to collect elution. 20% elution was concentrated and dried, to provide the extract sample 1 (Content:chlorogenic acid = 23.04%,geniposidic acid = 1.29%,aucubin = 3.54%); 60% elution was concentrated and dried, to provide the extract sample 2 (Content:chlorogenic acid = 10.21%,geniposidic acid = 1.03%,aucubin = 1.24%).

### Example 4 Preparation of eucommia leaf extract according to the present invention

The eucommia leaves were extracted twice with water (using 12 times the volume of leaves) at 60 °C, 2 h for each time. The extract was saturatedly absorbed by AB-8 macroporous absorption resins, and successively eluted with gradient ethanol of 10% and 90%, to collect elution. 10% elution was concentrated and dried, to provide the extract sample 1 (Content: chlorogenic acid = 39.11%, geniposidic acid = 10.33%, aucubin = 10.68%); 90% elution was concentrated and dried, to provide the extract sample 2 (Content: chlorogenic acid = 9.38%, geniposidic acid = 1.11%, aucubin = 2.31 %).

### Example 5 Preparation of medicaments according to the present invention (Tablets)

**Formula**

| | |
|---|---|
| Eucommia leaf extract | 200g |
| Starch | 100g |
| Hydroxypropylcellulose | 100g |
| Povidone | 15g |
| Talc powder | 5g |
| Total | 1000 tablets |

According to the formula, the extract sample 2 prepared in Example 1 was weighed, and mixed with starch and hydroxypropylcellulose, then sieved. The solution of povidone in EtOHwas added to the mixture, followed by wet granulation, drying, breaking. Talc powder was added for pressing tablet.

### Example 6 Preparation of medicaments according to the present invention (Granules)

**Formula**

| | |
|---|---|
| Eucommia leaf extract | 800g |
| Starch | 100g |
| Hydroxypropylcellulose | 100g |
| Magnesium stearate | 5g |
| Total | 1000 bags |

According to the formula, the extract sample 1 prepared in Example 1 was weighed, and mixed with starch and hydroxypropylcellulose, then sieved. EtOHwas added to the mixture, followed by wet granulation, drying, breaking. Magnesium stearate was added, and then packed in bags.

### Example 7 Preparation of medicaments according to the present invention (Pulvis)

**Formula**

| | |
|---|---|
| Eucommia leaf extract | 500g |
| Mannitol | 200g |
| Total | 1000 bags |

According to the formula, the extract sample 1 prepared in Example 2 was weighed, to which was added mannitol, followed by sieving and mixing, then bagging.

### Example 8 Preparation of medicaments according to the present invention (Pills)

**Formula**

| | |
|---|---|
| Eucommia leaf extract | 200g |
| Starch | 100g |
| Hydroxypropylcellulose | 100g |
| Carboxymethylstarch sodium | 50g |
| Total | 1000 pills |

According to the formula, the extract sample 2 prepared in Example 2 was weighed, and mixed with starch and hydroxypropylcellulose, then sieved. Carboxymethylstarch sodium was added to the mixture to prepare pills, followed by drying and breaking. Magnesium stearate was added, and then packed in bags.

### Example 9 Preparation of medicaments according to the present invention (oral solution)

**Formula**

| |
|---|
| Eucommialeaf extract200g |
| Water for injection appropriate amount |
| total 1000 bottles |

According to the formula, the extract sample 1 prepared in Example 3 was weighed, to which water for injection was added to adjust the volume, and then bottled.

### Example 10 Preparation of medicaments according to the present invention (Capsules)

**Formula**

| | |
|---|---|
| Eucommialeaf extract | 200g |
| Starch | 100g |
| Total | 1000 granules |

According to the formula, the extract sample 2 prepared in Example 3 was weighed, to which starch was added for granulating, drying, breaking, and then packed in capsules.

Hereinafter, the beneficial effect was further elucidated by pharmacodynamic experiment.

### Experimental example 1 Pharmacodynamic test of the eucommia leaf extract according to the present invention for treatment of psoriasis (psoriasis vulgarismodel)

### 1. Materials

### 1) Animals

50 6-8 weeks SCID mice, with body weight of 18-22 g, are provided by the Experimental Animal Center of Sichuan University. Animal grade: first class; license number: No. 10.

### 2) Drugs

Test drug 1 (Extract sample 1 in Example 1);
Test drug 2 (Extract sample 2 in Example 1);
Test drug 3 (Extract sample 1 in Example 2);

Acitretin A capsule is purchased from ChongqingHuaPont Pharm. Co., Ltd, with a batch number: 2013010.

### 2.Experimental methods

### 1) Establishing model and grouping

Healthy guinea pigs were divided into five groups using random digits table: (1) Model control group; (2) Positive control group; (3) Test drug 1 group; (4) Test drug 2 group; (5) Test drug 3 group.

### 2) Establishing animal model

Female and male SCID mice were separately fed for one week, and allowed to adapt growing environment. Patients with psoriasis vulgaris or plaque-type psoriasis were chosen (indentifying the informed consent). Dermatochalasis containing 2 cm × 2 cm psoriasis skin damage in patients was chosen to make a fusiform incision, and full-thickness of skin was cut and trimmed into free skin graft with a suitable thickness of 0.6 cm × 0.6 cm. Aseptic packaging was wrapped up with bandage macerated with normal saline, to prepare for transplantation. According to the body weight of SCID mice, 3% chloral hydrate (0.1 ml/10 g) was administrated, followed by skin preparation and unhair. Epidermis was scissored and cut into fascia layer, and the trimmed skin graft was sutured using 3-0 silk thread and subjected to pressure dressing with cotton ball soaking with normal saline. After transplantation of skin, mice were raised in separated cages, and all processes were finished within 2 h. After 10 days, skin damage arounding was injected with PBMC (1 × 10⁶/mouse) provided by transplantation supplier and treated with ConA. After two weeks, exterior packing was dismantled to observe the survival situation of skin and confirm the success of model establishment, and the successful model was used for further experiment.

### 3) Administration

Positive drug control group was given suspension of acitretin A capsule at 2.25mg/kg, and the amount of intragastric administration is all 1mL/100g for each time, twice a day; test drug 1, test drug 2, and test drug 3 were given at 1 mL/100g by intragastric administration, using a dosage of 100 mg/kg, once a day; the model group received equal amount of normal saline every day by intragastric administration. The administration was kept for 10 days.

### 4) Detection

After completion of administration, mice were sacrificed by cervical dislocation, and their transplanted skin was taken from the back and fixed in 10% formaldehyde (formaldedyde solution) for 24 h. Skin damage was trimmed into suitable size, dehydrated, embedded, sliced, and stained with HE. The change of inflammatory cells and so on in epidermis and dermis of transplanted skin damage were observed under optical microscope, and epidermic cell layers and epidermic thickness were counted under 200 times visual fields, to calculate numbers of lymphocytes and monocytes surrounding superficial layer blood vessels in dermis. Baker scores were calculated, and the difference in data of each group was compared.

Before sacrificing guinea pigs, blood was taken and centrifuged under 2000r/rain, to collect serum. Using ELISA kit, optical density was detected as instructions in kit. The results were calculated by standard curve, and the ratio of cAMP/cGMP was calculated according to the results.

### 5) Statistical analysis

All valueswere processed using SPSS13.0 software, and the experimental results were presented as x̅±s. p<0.05 was considered to be a statistical difference. The mean of multi-samples was compared using analysis of variance.

### 6) Baker scores

Modified Baker score method of experimental animals were as follows: parakeratosis in corneum layer scores 0.5-1.5 points (light-heavy); hyperkeratosis scores 1.5 point; cuticular process extension as rod scores 0.5-1.5 points (light-heavy); acanthosis scores 1.0 point; stratum granulosum thinning and disappearing scores 1.0 point; inflammatory cells infiltration in dermis scores 0.5-1.5 points (light-heavy); mastoid upward clubbing scores 1.0 point; telangiectasis scores 1.0 point.

### 3. Results

### (1) Baker scores, monocyte count, acanthocyte count of each group

Change of each group under light microscope are as follows: for positive control, middle degree of parakeratosis, spinous layer thickness being about 24-32 layers , epidermis presenting middle degree of wooden stick-like hyperplasia, local visible punctiform granular layer and hyperkeratinization, small amounts of monocyte infiltration surrounding blood vessels in dermis. For test drug 1 group,light degree of parakeratosis, spinous layer thickness being about 21-29 layers, epidermis presenting slight wooden stick-like hyperplasia, almost without punctiform granular layer, small amounts of monocyte infiltration surrounding blood vessels in dermis.For test drug 2 group, light degree of parakeratosis, spinous layerthickness being about 20-28 layers, epidermis presenting slight wooden stick-like hyperplasia, almost without punctiform granular layer, small amounts of monocyte infiltration surrounding blood vessels in dermis. For test drug 3 group, light degree of parakeratosis, spinous layer thickness being about 20-29 layers, epidermis presenting slight wooden stick-like hyperplasia, almost without punctiform granular layer, small amounts of monocyte infiltration surrounding blood vessels in dermis. For model control group, heavy degree of parakeratosis, spinous layer thickness being about 26-38 layers , epidermis presenting heavy degree of wooden stick-like hyperplasia, almost without punctiform granular layer, middle amounts of monocyte infiltration surrounding blood vessels in dermis.

**Table 1 Baker scores, monocyte count, acanthocyte count of each group (x̅±s, n=10)**

| Groups | Baker scores | Monocytes | Number of acanthocytes |
|---|---|---|---|
| Test drug 1 group | 5.02±1.02^{a} | 46.52±11.37^{a} | 24.69±5.77^{a} |
| Test drug 2 group | 4.46±0.85^{ab} | 38.09±6.09^{ab} | 21.11±5.27^{ab} |
| Test drug 3 group | 4.82±0.51^{ab} | 42.47±8.53^{ab} | 23.35±7.05^{ab} |
| Postive control group | 6.39±1.53^{a} | 66.40±9.28^{a} | 27.09±8.83^{a} |
| Model control group | 7.64±1.24 | 115.07±17.38 | 32.42±5.06 |

| | | | |
|---|---|---|---|
| Note: Compared with model control group, ^{a}P<0.01;compared with positive control group, ^{b}P<0.05. | | | |

Results showed that compared with model group, test drug 1 group, test drug 2 group and test drug 3 group showed significant difference (P<0.01); that compared with positive control group, test drug 2 group and test drug 3 group showed significant difference (P<0.05)

It can be seen that the eucommia leaf extract can improve Baker scores and reduce monocyte counts infiltrated in dermis of psoriasis animal model, suggesting the eucommia leaf extract of the present inventionhas a good therapeutic effect on psoriasis vulgaris. Amongst, test drug 2 is the best.

### (2) Effect on the content and the ratio of cAMP and cGMP in serum

**Table 2Effect on the content and the ratio of cAMP and cGMP in serum(x±s, n=10)**

| Groups | cAMP (pmol/ml) | cGMP (pmol/ml) | cAMP/cGMP |
|---|---|---|---|
| Blank control group | 28.54±1.65^{b} | 2.21±0.06^{b} | 12.91±1.38^{b} |
| Model group | 4.39±0.18^{a} | 4.01±0.13^{a} | 1.09±0.04^{a} |
| Positive control group | 10.22±0.73^{ab} | 3.38±0.20^{ab} | 3.02±0.08^{ab} |
| Test drug 1 group | 20.67±1.04^{abc} | 2.83±0.09^{abc} | 7.30±0.12^{abc} |
| Test drug 2 group | 22.02±1.39^{abc} | 2.92±0.12^{abc} | 7.54±0.27^{abc} |
| Test drug 3 group | 18.34±1.17^{abc} | 2.64±0.16^{abc} | 6.95±0.21^{abc} |

| | | | |
|---|---|---|---|
| Note: Compared with blank group, ^{a}P<0.01;Compared with model group, ^{b}P<0.01; compared with positive group, ^{c}P<0.05. | | | |

For guinea pig model with psoriasis,the content of cAMP in serum had an obvious decrease, while the content of cGMP in serum had an obvious increase (P<0.01). Compared with the model group, all of test drug groups and the positive control group had a significant increase in the content of cAMP(P<0.01), but an decrease in the content of cGMP(P<0.01), as well as the increase in the ratio of cAMP/cGMP(P<0.01); compared with the content of cGMP and the content of cAMP in positive control group, test drug 1 group, test drug 2 group and test drug 3 group showed a statistical significance (P<0.05), in which test drug 2 is the best.

### (3) Effect on VEGF levels in serum

**Table 3 Effect on VEGF levels in serum(x̅±s, n=10)**

| Groups | VEGF (pg/ml) |
|---|---|
| Blank control group | 72.29±5.56^{bc} |
| Model group | 138.37±8.81^{ac} |
| Positive control group | 93.40±6.54^{ab} |
| Test drug 1 group | 78.62±3.57^{abc} |
| Test drug 2 group | 74.94±4.01^{abc} |
| Test drug 3 group | 78.53±5.33^{abc} |

| | |
|---|---|
| Note: Compared with blank group, ^{a}P<0.01;Compared with model group, ^{b}P<0.01; Compared with positive group, ^{c}P<0.01. | |

For guinea pig model with psoriasis, VEGF was significantly higher than the normal group, indicating models were successfully made. After treatment, compared with the model group, all of the test drug groups and the positive control group showed a lower VEGF level (P<0.01), having a significant difference; VEGF in each test drug group is lower than that in the positive control group (P<0.01), having a significant difference, in which the test drug 2 (extract sample 2 in Example 1) is the best.

The eucommia leaf extract of the present invention can effectively improve the Baker scores of psoriasis model, increase the content of cAMP and cGMP in serum, and decrease the level of VEGF in serum, indicating the eucommia leaf extract of the present invention can efficaciously treat psoriasis vulgaris (Yuanlin Liu et al, "Change of E-cad and VEGF levels in serum of patients with psoriasis vulgaris before and after treatment", Chinese Journal of Laboratory Diagnosis, 2008-9, 12(9), 1162; Fei Li, "The effect of calcium dibutyacyladenosinecyclophosphate combined with acitretin capsules on psoriasis vulgaris and influence of the treatment on serum levels of cAMP and cGMP",J DiagnTher Dermato-Venereol2010-10, 17(5)), and test drug 2 showed the best therapeutic effect.

### Example 2 Pharmacodynamic test of the eucommia leaf extract according to the present invention for treatment of psoriasis (psoriasis pustulosa)

### Experimental study:

### 1. Materials

### 1) Animals

Kunmin mice, with weight of 18-22 g, are provided by Experimental Animal Center of Sichuan University. Animal grade: first class; license number: No. 10.

### 2) Drugs

Test drug 1 (Extract sample 1 in Example 3);
Test drug 2 (Extract sample 2 in Example 3);

### 2. Experimental method

### 1) Grouping of animals

Kunmin mice were randomly divided into 3 groups. For each test drug group, each mouse received drugs at 1 mL/100 g by gavage, and the administration dosage was 100 mg/kg; for the model control group, equal amount of normal saline was administrated to mice by gavage. Following time points were observed, i.e. before administration (day 0), days 1, 2, 4, 6 after administration.

### 2) Experimental method

After a piece of sterile gelatin sponge (1 cm²) was immersed in 0.75% zymosan A for 30 min, it was placed in peritoneal cavity of mice by surgery, and mice received standard diet. 24 hours after surgery, 5 mice of same phasic point were decapitated, and the gelatin sponges were taken out and stored in 0.5 ml cell eluant for culturing 30 min at 37 °C, respectively. The gelatin sponges were washed with 0.01 mol/LPBS 0.5 ml thrice, respectively, and the eluant was collected, passed through 40 µm nylon screen cloth, centrifuged, and the volume was 0.5 ml. Cells in above eluants were counted by automatic blood cell counter, and the amount of polymorphonuclear leukocytes (PMN) was read.

### 3. Results

**Table 4PMN transport numbers at different phasic points in test drug groups (×10⁹/L)**

| Groups | Day 0 | Day 1 | Day 2 | Day 4 | Day 6 |
|---|---|---|---|---|---|
| Blank control group | 9.56±1.12 | 11.38±2.07 | 9.45±2.96 | 8.30±1.11 | 8.47±2.21 |
| Test drug 1 group | 10.23±4.47 | 4.04±0.86* | 2.02±0.74Δ | 1.65±0.53Δ | 1.07±0.44Δ |
| Test drug 2 group | 9.47±2.12 | 2.82±1.41Δ | 1.77±0.53Δ | 1.08±0.58Δ | 1.02±0.43Δ |

| | | | | | |
|---|---|---|---|---|---|
| Note: compared with the blank group, *P<0.05; ΔP<0.01; | | | | | |

Results showed that from day 2, PMN in test drug 1 group and test drug 2 group obviously decreased, with a significant difference compared with the blank group, indicating the eucommia leaf extract had therapeutic effects on psoriasis pustulosa (Shengping Chen, Arotinoidethylester in psoriasis: clinical observation and laboratory inventigation, Master's degree at Third Military Medical University, 2002), in which the effect of test drug 2 group (extract sample 2 in Example 3) was relatively better.

In summary, the eucommia leaf extract of the present invention can effectively treat psoriasis, especially having a definite curative effect on psoriasis vulgaris or psoriasis pustulosa, which provided a new drug for clinical treatment of psoriasis.

## Claims

1. Aeucommia leaf extract, **characterized in that** it is the water or ethanolic extract of eucommia leaves, and in which the content of chlorogenic acid is not less than 9%, the content of geniposidic acid is not less than 1%, and the content of aucubin is not less than 1%.

2. The eucommia leaf extract according to claim 1, **characterized in that** in the eucommia leaf extract, the content of chlorogenic acid is 9.38%-39.11%, the content of geniposidic acid is 1.03%∼10.33%, and the content of aucubin is 1.17%-10.68%.

3. The eucommia leaf extract according to claim 2, **characterized in that** in the eucommia leaf extract, the content of chlorogenic acid is 10.21%-32.37%, the content of geniposidic acid is 1.03%-9.38%, and the content of aucubin is 1.17%-9.56%.

4. The eucommia leaf extract according to claim 3, **characterized in that** in the eucommia leaf extract, the content of chlorogenic acid is 10.21% or 32.37%, the content of geniposidic acid is 1.03% or 2.24%, and the content of aucubin is 1.24% or 2.07%.

5. The eucommia leaf extract according to any one of claims 1-4, **characterized in that** the eucommia leaf extract is prepared by the following method:
The eucommia leaves are extracted with water or 30-80% ethanol for 1-2 times, 1-2 h for each time. The extracting solution or its concentrated solution is absorbed by polystyrene-type macroporous absorption resins, followed by elution with gradient ethanol of 10-90%, and the eluent is collected, combined, concentrated and dried, to provide the extract.

6. The eucommia leaf extract according to claim 5, **characterized in that** the eucommia leaf extract is prepared by the following method:
The eucommia leaves are extracted twice with 60% ethanol at 70 °C, each time with 15 times the volume, 1 h for each time. The extract is combined and concentrated to recovery ethanol, and the concentrated solution is saturatedly absorbed by D-101 macroporous absorption resins, and eluted with gradient ethanol of 10%, 30%, 70%, and 90%, to collect elution, 10%elution and 30% elution are combined, or 70%elution and 90% elution are combined, and then concentrated and dried, respectively, to provide the extract;
Alternatively, the eucommia leaves are extracted twice with 30% ethanol (using 12 times the volume of leaves) at 60 °C, 2 h for each time. The extract is combined and concentrated to recovery ethanol, and the concentrated solution is saturatedly absorbed by HPD-100macroporous absorption resins, and eluted with gradient ethanol of 10%, 30%, 70%, and 90%, to collect elution. 70% EtOH elution and 90% EtOH elutionare combined, concentrated and dried, to provide the extract, or 30% EtOH elution is concentrated and dried, to provide the extract;
Alternatively, the eucommia leaves are extracted once with water (using 20 times the volume of leaves) at 80 °C, 1.5 h for each time. The extract is saturatedly absorbed by XAD-16macroporous absorption resins, and successively eluted with gradient ethanol of 20% and 60%, to respectively collect 20% elution and 60% elution, which are concentrated and dried, to provide the extract;
Alternatively, the eucommia leaves are extracted twice with water (using 12 times the volume of leaves) at 60 °C, 2 h for each time. The extract is saturatedly absorbed by AB-8 macroporous absorption resins, and successively eluted with gradient ethanol of 10% and 90%, to respectively collect 10% elution and 90% elution, which are concentrated and dried, to provide the extract.

7. The preparation method of eucommia leaf extract according to any one of claims1-6, **characterized in that** the method includes the following steps: the eucommia leaves are extracted with water or 30-80% EtOH for 1-2 times, 1-2 h for each time, and the extraction or its concentrated solution are absorbed by polystyrene-type macroporous absorption resins, followed by elution with gradient ethanol of 10-90%, and the eluent is collected, combined, concentrated and dried, to provide the extract.

8. The method according to claim 7, **characterized in that** the method includes the following steps:
The eucommia leaves are extracted twice with 60% ethanol (using 15 times the volume of leaves) at 70 °C, 1 h for each time. The extract is combined and concentrated to recovery ethanol, and the concentrated solution is saturatedly absorbed by D-101 macroporous absorption resins, and successively eluted with gradient ethanol of 10%, 30%, 70%, and 90%, to collect elution. 10% elution and 30% elution are combined, and 70% elution and 90% elution are combined, and then concentrated and dried, respectively, to provide the extract;
Alternatively, the eucommia leaves are extracted twice with 30% ethanol (using 12 times the volume of leaves) at 60 °C, 2 h for each time. The extract is combined and concentrated to recovery ethanol, and the concentrated solution is saturatedly absorbed by HPD-100 macroporous absorption resins, and eluted with gradient ethanol of 10%, 30%, 70%, and 90%, to collect elution. 70% EtOH elution and 90% EtOH elution are combined, concentrated and dried, to provide the extract, or 30% EtOH elution is concentrated and dried, to provide the extract;
Alternatively, the eucommia leaves are extracted once with water (using 20 times the volume of leaves) at 80 °C, 1.5 h for each time. The extract is saturatedly absorbed by XAD-16 macroporous absorption resins, and successively eluted with gradient ethanol of 20% and 60%, to respectively collect 20% elution and 60% elution, which were concentrated and dried, to provide the extract;
Alternatively, the eucommia leaves are extracted twice with water (using 12 times the volume of leaves) at 60 °C, 2 h for each time. The extract is saturatedly absorbed by AB-8 macroporous absorption resins, and successively eluted with gradient ethanol of 10% and 90%, to respectively collect 10% elution and 90% elution, which are concentrated and dried, to provide the extract.

9. Uses of the eucommia leaf extract according to any one of claims 1-6 in the preparation of medicaments for treatment of psoriasis.

10. Uses according to claim 9, **characterized in that** said medicaments are those treating psoriasisvulgaris and psoriasis pustulosa.

11. A medicament for treatment of psoriasis, **characterized in that** it is prepared using the eucommia leaf extract according to any one of claims 1-6 as active ingredient, together with pharmaceutically acceptable adjuvants or auxiliary ingredients.

12. The medicament according to claim 11, **characterized in that** said medicament is oral preparations, external preparations.

13. The medicament according to claim 12, **characterized in that** said oral preparations are tablets, granules, pulvis, pills, oral liquid, and capsules.
